(19) Europäisches Patentamt
European Patent Office
Office européen des brevets

(11) Veröffentlichungsnummer: **0 349 859 A2**

# (12) EUROPÄISCHE PATENTANMELDUNG

(21) Anmeldenummer: 89111526.3

(22) Anmeldetag: 24.06.89

(51) Int. Cl.[4]: **C07D 487/08 , //(C07D487/08, 241:00,241:00)**

(30) Priorität: 08.07.88 DE 3823160

(43) Veröffentlichungstag der Anmeldung: 10.01.90 Patentblatt 90/02

(84) Benannte Vertragsstaaten: BE DE FR GB NL

(71) Anmelder: BASF Aktiengesellschaft
Carl-Bosch-Strasse 38
D-6700 Ludwigshafen(DE)

(72) Erfinder: Hoelderich, Wolfgang, Dr.
Mannheimer Strasse 18 c
D-6710 Frankenthal(DE)

## (54) Verfahren zur Herstellung von 1,4-Diazabicyclo-2,2,2-octanen.

(57) Verfahren zur Herstellung von 1,4-Diazabicyclo-2,2,2-octanen der allgemeinen Formel I

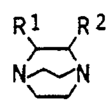

(I),

in der $R^1$ und $R^2$ unabhängig voneinander für Wasserstoff, $C_1$- bis $C_4$-Alkyl oder $C_2$- bis $C_4$-Alkenyl stehen, indem man Piperazine der allgemeinen Formel II

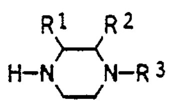

(II),

in der $R^1$ und $R^2$ die oben genannten Bedeutungen haben und $R^3$ für Wasserstoff oder $C_1$- bis $C_8$-Alkyl steht, in Gegenwart von Heterogenkatalysatoren bei Temperaturen von 50 bis 600° C und Drücken von 0,01 bis 50 bar, gegebenenfalls unter Mitverwendung eines geeigneten Lösungs- oder Verdünnungsmittels, umsetzt.

EP 0 349 859 A2

## Verfahren zur Herstellung von 1,4-Diazabicyclo-2,2,2-octanen

Die vorliegende Erfindung betrifft ein neues und verbessertes Verfahren zur Herstellung von 1,4-Diazabicyclooctanen durch Umsetzung von Piperazinen in Gegenwart von Heterogenkatalysatoren bei erhöhten Temperaturen.

1,4-Diazabicyclo-2,2,2-octane sind nach US-A-3,772,293 aus Piperazin und Ethylenoxid, nach DE-A-24 42 929 aus N,N′-Dihydroxyethylpiperazin unter Abspaltung von Glykol, nach EP-A-158 319, DE-A-24 34 913, US-A-3,166,588 und EP-A-111,928 z.B. aus N-Hydroxyethyl- oder N-Aminoethyl-piperazin oder nach US-A-3,883,533 aus 1,4-Bis-(formyl)-piperazin und Ethylenglykol, erhältlich.

Als Heterogenkatalysatoren wurden Phosphate des Ca, Sr, Ba, Zn, La, Al, Co, Ni oder Ce oder Aluminiumsilikatzeolithe des Pentasiltyps, A-Zeolithe oder Silica-Alumina-Crackkatalysatoren verwendet.

Die bekannten Verfahren liefern unbefriedigende Ausbeuten und/oder Selektivitäten und/oder es treten unerwünschte, schwer oder unvollständig abtrennbare Nebenprodukte auf.

Der Erfindung lag daher die Aufgabe zugrunde, einen besseren Zugang zu den 1,4-Diazabicyclo-2,2,2-octanen zu finden und den Nachteilen der bekannten Verfahren abzuhelfen.

Demgemäß wurde ein neues und verbessertes Verfahren zur Herstellung von 1,4-Diazabicyclo-2,2,2-octanen gefunden, welches dadurch gekennzeichnet ist, daß man Piperazine, die an ihren Stickstoffatomen Wasserstoff oder eine Alkylgruppe tragen, in Gegenwart von Heterogenkatalysatoren bei Temperaturen von 50 bis 600 °C und Drücken von 0,01 bis 50 bar, gegebenenfalls unter Mitverwendung eines geeigneten Lösungs- oder Verdünnungsmittels, umsetzt.

Die 1,4-Diazabicyclo-2,2,2-octane I sind nach folgender Methode erhältlich:

Die Umsetzung erfolgt durch thermische Behandlung von Piperazinen an einem Heterogenkatalysator.

Die Reaktion kann sowohl in der Flüssigphase als auch in der diskontinuierlichen oder vorzugsweise kontinuierlichen Gasphase bei 50 bis 600 °C und 0,01 bis 50 bar durchgeführt werden.

Die Flüssigphasenreaktion kann beispielsweise als Suspensions-, Riesel-oder Sumpfreaktion bei Temperaturen von 50 bis 200 °C und Drücken von 0,5 bis 20 bar, vorzugsweise bei Temperaturen von 70 bis 170 °C und Drücken von 1 bis 5 bar durchgeführt werden.

Die bevorzugte Gasphasenreaktion kann beispielsweise bei Temperaturen von 100 bis 600 °C, vorzugsweise bei 150 bis 500 °C und Drücken von 0,1 bis 50 bar, besonders bevorzugt bei 200 bis 400 °C und Drücken von 0,5 bis 5 bar durchgeführt werden. Bei der Umsetzung in der Gasphase hält man vorteilhaft eine Katalysatorbelastung von 0,1 bis 20, insbesondere von 0,5 bis 5 g Ausgangsstoff der Formel II je g Katalysator und Stunde ein (WHSV = g Einsatzgemisch/g Katalysator und Stunde). Die Gasphasenreaktion kann in einem Festbett oder in einem Wirbelbett ausgeführt werden.

Nach der Umsetzung werden die entstandenen Produkte durch übliche Verfahren, z.B. durch Destillation aus dem Reaktionsgemisch isoliert; nichtumgesetzte Ausgangsstoffe werden gegebenenfalls in die Umsetzung zurückgeführt.

Bevorzugt werden gasförmigen Reaktionsprodukte sofort in eine Trennung eingebracht und z.B. in einer Fraktionierkolonne in ihre Einzelkomponenten zerlegt.

Die Piperazine II können ohne, bevorzugt aber mit Lösungs- oder Verdünnungsmitteln eingesetzt werden. Als solche eignen sich organische Lösungs- oder Verdünnungsmittel wie aliphatische oder alicyclische Alkohole, z.B. $C_1$- bis $C_{12}$-Alkanole, bevorzugt $C_1$- bis $C_6$-Alkohole, besonders bevorzugt Methanol und Ethanol, $C_5$- bis $C_{20}$-Cycloalkanole, bevorzugt Cyclohexanol, Diole wie $C_1$- bis $C_{12}$-Hydroxyalkanole, bevorzugt $C_1$- bis $C_4$-Hydroxyalkanole, wie Ethylenglykol, Butandiole und Hexandiole, acyclische oder cyclische Ether mit 2 bis 12 Kohlenstoffatomen, wie Dimethylether, Diethylether, Dibutylether, Dipropylether, Dipentylether oder deren Isomere und THF, Pyran oder Lactone, Polyether, wie Monoglyme, Diglyme, Triglyme, aromatische oder aliphatischen Kohlenwasserstoffen wie Benzol, Toluol, Xylol, Pentan, Cyclopentan, Hexan und Petrolether oder deren Gemische und besonders auch Wasser oder wäßrige organische Lösungs- oder Verdünnungsmittel der oben genannten Art.

Die Konzentration der Piperazine II in Wasser kann zwischen 5 und 95 Gew.%, bevorzugt zwischen 20 und 60 Gew.%, liegen. Das Molverhältnis von organischen Lösungs- oder Verdünnungsmittel zum Piperazin II kann 0,1:1 bis 50:1, bevorzugt 1:1 bzw. 15:1 betragen.

Als Verdünnungsmittel für die Gasphase eignen sich auch Inertgase wie Stickstoff oder Argon, in einigen Fällen auch Sauerstoff.

Die Substituenten $R^1$ und $R^2$ in den Formeln I und II haben unabhängig voneinander folgende Bedeutungen:

- Wasserstoff,
- unverzweigtes oder verzweigtes $C_1$- bis $C_4$-Alkyl wie Methyl, Ethyl, n-Propyl, iso-Propyl, n-Butyl, iso-Butyl,

sec.-Butyl und tert.-Butyl, bevorzugt Methyl und Ethyl,

- unverzweigtes oder verzweigtes $C_2$- bis $C_4$-Alkenyl wie Vinyl, Allyl und Butenyl.

Der Substituent $R^3$ in Formel II bedeutet Wasserstoff oder $C_1$- bis $C_8$-Alkyl wie Methyl, Ethyl, n-Propyl, iso-Propyl, n-Butyl, iso-Butyl, sec.-Butyl, tert.-Butyl, Pentyl, Hexyl und Octyl.

Als Katalysatoren für das erfindungsgemäße Verfahren werden feste Heterogenkatalysatoren, bevorzugt saure feste Heterogenkatalysatoren, eingesetzt.

Als Katalysatoren für das erfindungsgemäße Verfahren werden insbesondere acide zeolithische Katalysatoren eingesetzt. Zeolithe sind kristalline Aluminosilikate, die eine hochgeordnete Struktur mit einem starren dreidimensionalen Netzwerke von $SiO_4$- und $AlO_4$-Tetraedern besitzen, die durch gemeinsame Sauerstoffatome verbunden sind. Das Verhältnis der Si- und Al-Atome zu Sauerstoff beträgt 1:2 (siehe Ullmanns Encyclopädie d. techn. Chemie, 4. Auflage, Band 24, Seite 575 (1983)). Die Elektrovalenz der Aluminium enthaltenden Tetraeder ist durch Einschluß von Kationen in den Kristall, z.B. eines Alkali- oder Wasserstoffions ausgeglichen. Ein Kationenaustausch ist möglich. Die Räume zwischen den Tetraedern sind vor der Dehydration durch Trocknen bzw. Calcinieren von Wassermolekülen besetzt.

In den Zeolithen können anstelle von Aluminium auch andere Elemente wie B, Ga, Fe, Cr, V, As, Sb, Bi oder Be oder deren Gemische in das Gitter eingebaut werden, oder das Silicium kann durch ein vierwertiges Element wie Ge, Ti, Zr, Hf ersetzt werden.

Entsprechend ihrer Struktur werden Zeolithe in verschiedene Gruppen unterteilt (siehe Ullmanns Encyclopädie d. techn. Chemie, 4. Aufl., Bd. 24, S. 575 [1983]). So bilden bei der Mordenit-Gruppe Ketten oder bei der Chabasit-Gruppe Schichten aus Tetraedern die Zeolith-Struktur, während sich bei der Faujasit-Gruppe die Tetraeder zu Polyedern ordnen, z.B. in Form eines Kubooktaeders, der aus Vierringen bzw. Sechsringen aufgebaut ist. Je nach Verknüpfung der Kubooktaeder, wodurch unterschiedlich große Hohlräume und Poren entstehen, unterscheidet man in Zeolithe vom Typ A, L, X oder Y.

Für das erfindungsgemäße Verfahren in Betracht kommende Katalysatoren sind Zeolithe aus der Mordenit-Gruppe oder engporige Zeolithe vom Erionit- bzw. Chabasit-Type oder Zeolithe vom Faujasit-Typ, z.B. Y-, X- oder L-Zeolithe. In diese Gruppe von Zeolithen gehören auch die sogenannten "ultrastabilen" Zeolithe des Faujasittyps, d.h. dealuminierte Zeolithe. Verfahren zur Herstellung solcher Zeolithe sind beschrieben in "catalysis by Zeolites", Band 5 aus "studies in Surface Science and Catalysis", ed. B. Imelik et. al. Elsevier Scientific Publishing Comp., 1980, S. 203 und "Crystal Structures of Ultra-stable Faujasites", Advances in Chemistry, Series Nr. 101, American Chemical Society Washington, DC, S. 226ff (1971) und in US-PS 4 512 961.

Besonders vorteilhaft sind Zeolithe vom Pentasiltyp. Diese haben als Grundbaustein einen aus $SiO_4$-Tetraedern aufgebauten Fünfring gemeinsam. Sie sind durch ein hohes $SiO_2/Al_2O_3$-Verhältnis gekennzeichnet sowie durch Porengrößen, die zwischen denen der Zeolithe vom Typ A und denen vom Typ X oder Y liegen (vgl. Ullmanns Encyclopädie d. techn. Chem., 4. Auf., Bd. 24, 1983).

Diese Zeolithe können unterschiedliche chemische Zusammensetzung aufweisen. Es handelt sich hierbei um Alumino-, Boro-, Eisen-, Beryllium-, Gallium-, Chrom-, Arsen, Antimon- und Wismutsilikatzeolithode oder deren Gemische sowie Alumino-, Boro-, Gallium- und Eisengermanatzeolithe oder deren Gemische. Insbesondere eignen sich die Alumino-, Boro- und Eisensilikatzeolithe des Pentasiltyps für das erfindungsgemäße Verfahren. Der Aluminosilikatzeolith wird z.B. aus einer Aluminiumverbindung, vorzugsweise $Al(OH)_3$ oder $Al_2(SO_4)_3$ und einer Siliciumkpmponente, vorzugsweise hochdispersem Siliciumdioxid in wäßriger Aminlösung, insbesondere in Polyaminen wie 1,6-Hexandiamin- oder 1,3-Propandiamin oder Triethylentetramin-Lösung mit oder insbesondere ohne Alkali- oder Erdalkalizusatz bei 100 bis 200 °C unter autogenem Druck hergestellt. Auch gehören hierzu die isotaktischen Zeolithe nach EP 34 727 und EP 46 504. Die erhaltenen Aluminosilikatzeolithe enthalten je nach Wahl der Einsatzstoffmengen ein $SiO_2/Al_2O_3$-Verhältnis von 10 bis 40 000. Auch lassen sich derartige Aluminosilikatzeolithe in etherischem Medium wie Diethylenglykoldimethylether, in alkoholischem Medium wie Methanol bzw. 1,4-Butandiol oder in Wasser synthetisieren.

Der Borosilikatzeolith wird z.B. bei 90 bis 200 °C unter autogenem Druck synthetisiert, indem man eine Borverbindung, z.B. $H_3BO_3$, mit einer Siliciumverbindung, vorzugsweise hochdispersem Siliciumdioxid in wäßriger Aminlösung, insbesondere in 1,6-Hexandiamin- oder 1,3-Propandiamin- oder Triethylentetramin-Lösung mit und insbesondere ohne Alkali- oder Erdalkalizusatz zur Reaktion bringt. Auch gehören hierzu die isotaktischen Zeolithe nach EP 34 727 und EP 46 504. Solche Borosilikatzeolithe können ebenfalls hergestellt werden, wenn man die Reaktion statt in wäßriger Aminlösung, z.B. in Diethylenglykoldimethylether oder in alkoholischer Lösung, z.B. 1,6-Hexandiol durchführt.

Den Eisensilikatzeolith erhält man z.B. aus einer Eisenverbindung, vorzugsweise $Fe(SO_4)_3$ und einer Siliciumverbindung, vorzugsweise hochdispersem Siliciumdioxid in wäßriger Aminlösung, insbesondere 1,6-Hexandiamin, mit oder ohne Alkali- oder Erdalkalizusatz bei 100 bis 220 °C unter autogenem Druck.

Zu den verwendbaren siliciumreichen Zeolithen ($SiO_2/Al_2O_3$ ≧10) gehören auch die sog. ZSM-Typen, Ferrierit, NU-1 und Silicalit ® (ein Molekularsieb, ein sog. Silica Polymorph).

Die so hergestellten Alumino-, Boro- und Eisensilikatzeolithe können nach ihrer Isolierung, Trocknung bei 100 bis 160 °C, vorzugsweise 110 °C und Calcinierung bei 450 bis 550 °C, vorzugsweise 500 °C, mit einem Bindemittel im Verhältnis 90 : 10 bis 40 : 60 Gew.% zu Strängen oder Tabletten verformt werden. Als Bindemittel eignen sich diverse Aluminiumoxide, bevorzugt Boehmit, amorphe Aluminosilikate mit einem $SiO_2/Al_2O_3$-Verhältnis von 25 : 75 bis 90 : 5, bevorzugt 75 : 25, Siliciumdioxid, bevorzugt hochdisperses $SiO_2$, Gemische aus hochdispersem $SiO_2$ und hochdispersem $Al_2O_3$, $TiO_2$, $ZrO_2$ sowie Ton. Nach der Verformung werden die Extrudate oder Preßlinge bei 110 °C/16 h getrocknet und bei 500 °C/16 h calciniert.

Man erhält auch vorteilhafte Katalysatoren, wenn der isolierte Alumino-bzw. Borosilikatzeolith direkt nach der Trocknung verformt wird und erst nach der Verformung einer Calcinierung unterworfen wird. Die hergestellten Alumino- und Borosilikatzeolithe können in reiner Form, ohne Binder, als Stränge oder Tabletten eingesetzt werden, wobei als Verstranungs- oder Peptisierungshilfsmittel z.B. Ethylcellulose, Stearinsäure, Kartoffelstärke, Ameisensäure, Oxalsäure, Essigsäure, Salpetersäure, Ammoniak, Amine, Silikoester und Graphit oder deren Gemische verwendet werden.

Liegt der Zeolith aufgrund der Art seiner Herstellung nicht in der katalytisch aktiven, aciden H-Form, sondern z.B. in der Na-Form, dann kann diese durch Ionenaustausch z.B. mit Ammoniumionen und anschließende Calcinierung oder durch Behandlung mit Säuren vollkommen oder partiell in die gewünschte H-Form überführt werden.

Wenn bei der erfindungsgemäßen Verwendung der zeolithischen Katalysatoren eventuell eine durch Koksabscheidung bedingte Desaktivierung eintritt, empfiehlt es sich, die Zeolithe durch Abbrennen der Koksablagerung mit Luft oder mit einem Luft/$N_2$-Gemisch bei 400 bis 550 °C, bevorzugt regenerieren. Die Zeolithe erhalten dadurch ihre Anfangsaktivität zurück.

Durch partielle Verkokung (pre-coke) ist es möglich, die Aktivität des Katalysators für ein Selektivitätsoptimum des gewünschten Reaktionsproduktes einzustellen.

Um eine möglichst hohe Selektivität, hohen Umsatz sowie lange Standzeiten zu erreichen, kann es vorteilhaft sein, die Zeolithe zu modifizieren. Eine geeignete Modifizierung der Katalysatoren besteht z.B. darin, daß man den unverformten oder verformten Zeolithen mit Metallsalzen durch einen Ionenaustausch oder durch Imprägnierung dotiert. Als Metalle werden Alkalimetalle wie Li, Cs, K, Erdalkalimetalle wie Mg, Ca, Sr, Metalle der 3., 4 und 5. Hauptgruppe wie Al, Ga, Ge, Sn, Pb, Bi, Übergangsmetalle der 4. - 8. Nebengruppe wie Ti, Zr, V, Nb, Cr, Mo, W, Mn, Re, Fe, Ru, Os, Co, Rh, Sr, Ni, Pd, Übergangsmetalle der 1. und 2. Nebengruppe wie Cu, Ag, Zn, Seltene Erdmetalle wie La, Ce, Pr, Nd, kFr, Yb und U eingesetzt.

Zweckmäßigerweise führt man die Dotierung so durch, daß man z.B. den verformten Zeolithen in einem Steigrohr vorlegt und bei 20 bis 100 °C z.B. eine wäßrige oder ammoniakalische Lösung eines Halogenids oder eines Nitrats der voranbeschriebenen Metalle überleitet. Ein derartiger Ionenaustausch kann z.B. an der Wasserstoff-, Ammonium- und Alkaliform des Zeolithen vorgenommen werden. Eine weitere Möglichkeit der Metallaufbringung auf den Zeolithen ist gegeben, indem man das zeolithische Material z.B. mit einem Halogenid, einem Nitrat oder einem Oxid der voranbeschriebenen Metalle in wäßriger, alkoholischer oder ammoniakalischer Lösung imprägniert. Sowohl an einem Ionenaustausch als auch an eine Imprägnierung schließt sich zumindest eine Trocknung, wahlweise eine abermalige Calcinierung an.

Eine mögliche Ausführungsform besteht z.B. darin, daß man $Cu(NO_3)_2$ x3 $H_2O$ oder $Ni(NO_3)_2$ x 6 $H_2O$ oder $La(NO_3)_2$ x 6 $H_2O$ oder $Cs_2CO_3$ in Wasser löst. Mit dieser Lösung wird der verformte oder unverformte Zeolith eine gewisse Zeit, ca. 30 Minuten, getränkt. Die eventuell überstehende Lösung wird am Rotationsverdampferf von Wasser befreit. Danach wird der getränkte Zeolith bei ca. 150 °C getrocknet und bei ca. 550 °C calciniert. Dieser Tränkvorgang kann mehrmals hintereinander vorgenommen werden, um den gewünschten Metallgehalt einzustellen.

Auch ist es möglich, z.B. eine wäßrige $Ni(CO_3)_2$-Lösung oder ammoniakalische $Pd(NO_3)_2$-Lösung herzustellen und darin den reinen pulverförmigen Zeolithen bei 40 bis 100 °C unter Rühren ca. 24 h aufzuschlämmen. Nach Abfiltrieren, Trocknen bei ca. 150 °C und Calcinierung bei ca. 500 °C kann das so gewonnene zeolithische Material mit oder ohne Bindemittel zu Strängen, Pellets oder Wirbelgut weiterverarbeitet werden.

Ein Ionenaustausch des in der H-Form oder Ammonium-Form oder Alkali-Form vorliegenden Zeolithen kann so vorgenommen werden, daß man den Zeolithen in Strängen oder Pellets in einer Kolonne vorgelegt und darüber z.B. eine wäßrige $Ni(NO_3)_2$-Lösung oder ammoniakalische $Pd(NO_3)_2$-Lösung bei leicht erhöhter Temperatur zwischen 30 und 80 °C im Kreislauf 15 bis 20 h leitet. Danach wird mit Wasser ausgewaschen, bei ca. 150 °C getrocknet und bei ca. 550 °C calciniert. Bei manchen metalldotierten Zeoltihen z.B. Pd-, Cu-, Ni-dotierten Zeolithen ist eine Nachbehandlung mit Wasserstoff vorteilhaft.

Eine weitere Möglichkeit der Modifizierung besteht darin, daß man das zeolithische Material - verformt

oder unverformt - einer Behandlung mit Säuren wie Salzsäuren, Flußsäure und Phosphorsäure und/oder Wasserdampf unterwirft. Dabei geht man vorteilhaft z.B. so vor, daß man Zeolithe in Pulverform mit 1 n Phosphorsäure 1 Stunde bei 80°C behandelt. Nach der Behandlung wird mit Wasser gewaschen, bei 110°C/16 Stunden getrocknet und bei 500°C/20 Stunden calciniert. Nach einer anderen Arbeitsweise behandelt man Zeolithe vor oder nach ihrer Verformung mit Bindemitteln, z.B. 1 bis 3 Stunden bei Temperaturen von 60 bis 80°C mit einer 3 bis 25 gew.%igen, insbesondere 12 bis 20 gew.%igen wäßrigen Salzsäure. Anschließend wird der behandelte Zeolith mit Wasser gewaschen, getrocknet und bei 400°C bis 500°C calciniert.

Eine besondere Ausführungsform für die Säurebehandlung besteht darin, daß man das zeolithische Material vor seiner Verformung bei erhöhter Temperatur mit Flußsäure, die im allgemeinen als 0,001 n bis 2 n, vorzugsweise 0,05 n bis 0,5 n Flußsäure eingesetzt wird, behandelt, beispielsweise durch Erhitzen unter Rückfluß über einen Zeitraum von allgemeinen 0,5 bis 5, vorzugsweise 1 bis 3 Stunden. Nach Isolierung, z.B. durch Abfiltrieren und Auswaschen, des zeolithischen Materials wird dieses zweckmäßig, z.B. bei Temperaturen von 100 bis 160°C, getrocknet und bei Temperaturen von im allgemeinen 450 bis 600°C calciniert. Gemäß einer weiteren bevorzugten Ausführungsform für die Säurebehandlung wird das zeolithische Material nach einer Verformung mit Bindemittel bei erhöhter Temperatur, zweckmäßig bei Temperaturen von 50 bis 90°C, vorzugsweise 60 bis 80°C, über einen Zeitraum von 0,5 bis 5, vorzugsweise mit 12 bis 20 gew.%iger Salzsäure, behandelt. Anschließend wird das zeolithische Material im allgemeinen ausgewaschen und zweckmäßig, z.B. bei Temperaturen von 100 bis 160°C, getrocknet und bei Temperaturen von im allgemeinen 450 bis 600°C calciniert. Einer HF-Behandlung kann sich auch eine HCl-Behandlung anschließen.

Nach einer anderen Arbeitsweise lassen sich Zeolithe durch Aufbringung von Phosphorverbindungen, wie Trimethoxiphosphat, Trimethoxiphosphin, primärem, sekundärem oder tertiärem Natriumphosphat modifizieren. Hierbei werden die Zeolithe in Strang-, Tabletten- oder Wirbelgut-Form mit wäßriger $H_3PO_4$-Lösung getränkt, bei 110°C getrocknet und bei 500°C calciniert.

Weitere Katalysatoren für das erfindungsgemäße Verfahren sind Phosphate, insbesondere Aluminiumphosphate, Siliciumaluminiumphosphate, Siliciumeisenaluminiumphosphate, Cobaltaluminiumphosphate, Cerphosphat, Zirkonphosphate, Borphosphat, Eisenphosphat, Strontiumphosphate oder deren Gemische.

Als Aluminiumphosphat-Katalysatoren werden für das erfindungsgemäße Verfahren insbesondere unter hydrothermalen Bedingungen synthetisierte Aluminiumphosphate eingesetzt, die Zeolithstruktur besitzen.

Die unter hydrothermalen Bedingungen hergestellten Aluminiumphosphate sind z.B. APO-5, APO-9, APO-11, APO-12, APO-14, APO-21, APO-25, APO-31 und APO-33. Synthesen dieser Verbindungen sind in EP-A-132 708, US 4 310 440 und US 4 473 663 beschrieben.

Beispielsweise das $AlPO_4$-5 (APO-5) wird synthetisiert, indem man Orthophosphorsäure mit Pseudoboehmit (Catapal SB®) in Wasser homogen mischt; zu dieser Mischung Tetrapropylammoniumhydroxid gibt und danach bei ca. 150°C 20 bis 60 h unter autogenem Druck in einem Autoklaven umsetzt. Das abfiltrierte $AlPO_4$ wird getrocknet bei 100 bis 160°C und calciniert bei 450 bis 550°C.

$AlPO_4$-9 (APO-9) wird ebenfalls aus Orthophosphorsäure und Pseudoboehmit aber in wäßriger DABCO-Lösung (1,4-Diazabicyclo-2,2,2-octan) bei ca. 200°C unter autogenem Druck während 200 bis 400 h synthetisiert.

Die Synthese des $AlPO_4$-21 (APO-21) erfolgt aus Orthophosphorsäure und Pseudoboehmit in wäßriger Pyrrolidon-Lösung bei 150 bis 200°C unter autogenem Druck während 50 bis 200 h.

Die für das erfindungsgemäße Verfahren eingesetzten Siliciumaluminiumphosphate sind z.B. SAPO-5, SAPO-11, SAPO-31 UND SAPO-34. Die Synthese dieser Verbindung wird z.B. in EP 103 117, US 4 440 871 beschrieben. SAPO'S werden hergestellt durch Kristallisation aus wäßriger Mischung bei 100 bis 250°C und autogenem Druck während 2 h bis 2 Wochen, wobei die Reaktionsmischung aus einer Silicium-, Aluminium- und Phosphorkomponente in wäßrigen aminoorganischen Lösungen umgesetzt wird.

SAPO-5 beispielsweise wird durch Mischen von $SiO_2$ suspendiert in wäßriger Tetrapropylammoniumhydroxid-Lösung mit einer wäßrigen Suspension aus Pseudoboehmit und Orthophosphorsäure und anschließende Umsetzung bei 150 bis 200°C während 20 bis 200 h unter autogenem Druck in einem Rührautoklaven erhalten. Die Trocknung des abfiltrierten Pulvers erfolgt bei 110 bis 160°C und die Calcination bei 450 bis 550°C.

Als Phosphatkatalysatoren kann man bei dem Verfahren auch gefällte Aluminiumphosphate einsetzen. Beispielsweise wird ein derartiges Aluminiumphosphat hergestellt, indem 92 g Diammoniumhydrogenphosphat in 700 ml Wasser gelöst werden. Zu dieser Lösung wird 260 g $Al(NO_3)_3$ x $H_2O$ in 700 ml Wasser über 2 h zugetropft. Dabei wird der pH-Wert durch gleichzeitige Zugabe von 25 %iger $NH_3$-Lösung bei pH 8 gehalten. Der entstandene Niederschlag wird 12 h nachgerührt, dann abgesaugt und ausgewaschen. Er wird bei 60°C/16 h getrocknet.

Borphosphate für das erfindungsgemäße Verfahren lassen sich beispielsweise durch Mischen und Kneten von konzentrierter Borsäure und Phosphorsäure und durch anschließende Trocknung und Calcination in Inertgas-, Luft- und Dampfatmosphäre bei 250 bis 650 °C, vorzugsweise 300 bis 500 °C herstellen.

Auf diese Phosphate können auch Imprägnierung (Tränken und Aufsprühen) oder in manchen Fällen auch durch Ionenaustausch Modifizierungskomponen ten, wie voran bei den Zeolithen beschrieben, aufgebracht werden. Auch kann wie bei den Zeolithkatalysatoren eine Modifizierung mit Säuren erfolgen.

Geeignete saure Katalysatoren sind z.B. auch die sauer wirkenden Oxide von Elementen der III. und IV. Hauptgruppe sowie der IV. bis VI. Nebengruppe des periodischen Systems, insbesondere Oxide wie Siliciumdioxid in Form von Kieselgel, Kieselgur, Quarz, weiterhin Titandioxid, Zirkondioxid, Phosphoroxide, Vanadiumoxide, Nioboxide, Boroxide, Aluminiumoxide, Chromoxide, Molybdänoxide, Wolframoxide oder Bims oder Gemische dieser Oxide. Auch können diese Oxide durch Aufbringung von Modifizierungskomponenten, wie voran bei den Zeolithkatalysatoren beschrieben, dotiert werden. Die Behandlung mit Säuren, wie voran bei den Zeolithkatalysatoren beschrieben, ist ebenfalls eine eventuelle Möglichkeit der Modifizierung.

Auch mit Phosphorsäure oder Borsäure getränkte Katalysatoren können eingesetzt werden. Phosphorsäure oder Borsäure wird z.B. auf $SiO_2$-, $Al_2O_3$-, $TiO_2$- oder Bimsträger, z.B. durch Auftränken oder Versprühen aufgebracht. Ein phosphorsäurehaltiger Katalysator kann beispielsweise durch Auftränken von $H_3PO_4$- oder $NaH_2PO_4$- oder $Na_2HPO_4$-Lösung auf $SiO_2$ und anschließende Trocknung bzw. Calcination erhalten werden. Phosphorsäure kann aber auch mit Kieselgel zusammen in einem Sprühturm versprüht werden; danach schließt sich eine Trocknung und meist eine Calcination an. Phosphorsäure kann auch in einer Imprägniermühle auf das Trägermaterial aufgesprüht werden.

Aber auch basische Katalysatoren, wie MgO, CaO, Oxide der Lanthaniden, können Verwendung für das erfindungsgemäße Verfahren finden.

Die hier beschriebenen Katalysatoren können wahlweise als 2- bis 4-mm-Stränge oder als Tabletten mit 3 bis 5 mm Durchmesser oder als Splitt mit Teilchengrößen von 0,1 bis 0,5 mm oder als Wirbelkontakt eingesetzt werden.

Die folgenden Beispiele veranschaulichen die Erfindung:

Herstellung der Katalysatoren

Katalysator A

Der Borosilikatzeolith des Pentasil-Typs wird in einer hydrothermalen Synthese aus 640 g hochdispersem $SiO_2$, 122 g $H_3BO_3$, 8000 g einer wäßrigen 1,6-Hexandiamin-Lösung (Mischung 50:50 Gew.%) bei 170 °C unter autogenem Druck in einem Rührautoklaven hergestellt. Nach Abfiltrieren und Auswaschen wird das kristalline Reaktionsprodukt bei 100 °C/24 h getrocknet und bei 500 °C/24 h calciniert. Dieser Borosilikatzeolith setzt sich zusammen aus 94,2 Gew.% $SiO_2$ und 2,3 Gew.% $B_2O_3$.

Katalysator A wird erhalten, indem man den Borosilikatzeolith mit einem Verformungshilfsmittel zu 2-mm-Strängen verformt, bei 100 °C/16 h trocknet und bei 500 °C/24 h calciniert.

Katalysator B

Katalysator B wird hergestellt, indem man Katalysator A mit $Ce(NO_3)_2$ dotiert, bei 130 °C/2 h trocknet und bei 540 °C/2 h calciniert. Der Ce-Gehalt beträgt 1,8 Gew.%.

Katalysator C

Katalysator C wie Katalysator B hergestellt, jedoch mit einer wäßrigen Lösung aus Pd- und Ce-Nitrat anstatt Ce-Nitrat getränkt. Der Pd-Gehalt beträgt 1,3 Gew.%, der Ce-Gehalt 3,6 Gew.%.

Katalysator D

Katalysator D wird wie Katalysator B hergestellt, indem man jedoch statt mit Ce-Nitrat mit $Cs_2CO_3$

dotiert. Der Cs-Gehalt beträgt 0,2 Gew.%.

Katalysator E

Katalysator E wird wie Katalysator B hergestellt, indem man jedoch statt mit Ce-Nitrat mit $Cs_2CO_3$ dotiert. Der Cs-Gehalt beträgt 0,7 Gew.%.

Katalysator F

Katalysator F wird hergestellt wie Katalysator B, indem man jedoch statt mit Ce-Nitrat mit $Cs_2CO_3$ dotiert. Der Cs-Gehalt beträgt 2,3 Gew.%.

Katalysator G

Ein Aluminosilikatzeolith vom Pentasil-Typ wurde unter hydrothermalen Bedingungen bei autogenem Druck und 150 °C aus 65 g hochdispersem $SiO_2$, 20,3 g $Al_2(SO_4)_3$ x 18 $H_2O$ in 1 kg mit wäßrigem 1,6-Hexandiamin-Lösung (Mischung 50:50 Gew.%) in einem Rührautoklaven hergestellt. Nach Abfiltrieren und Auswaschen wurde das kristalline Reaktionsprodukt bei 110 °C/24 h getrocknet und bei 500 °C/24 h calciniert. Dieser Aluminosilikatzeolith enthielt 91,6 Gew.% $SiO_2$ und 4,6 Gew.% $Al_2O_3$.

Der Katalysator wurde mit einem Verformungshilfsmittel zu 2-mm-Strängen verformt, bei 110 °C/16 h getrocknet und bei 500 °C/24 h calciniert.

Katalysator H

Der Eisensilikatzeolith des Pentasil-Typs wird unter hydrothermalen Bedingungen bei autogenem Druck und 165 °C aus 273 g Wasserglas, gelöst in 253 g einer wäßrigen 1,6-Hexandiamin-Lösung (Mischung 50:50 Gew.%) und 31 g Eisensulfat, gelöst in 21 g 96 %iger Schwefelsäure und 425 g Wasser in einem Rührautoklaven während 4 Tagen synthetisiert. Der Zeolith wird abfiltriert, ausgewaschen, bei 110 °C/24 h getrocknet und bei 500 °C/24 h calciniert. Man erhält einen Eisensilikatzeolithen mit einem $SiO_2/Fe_2O_3$-Verhältnis von 17,7 und einen $Na_2O$-Gehalt von 1,2 Gew.%. Der Katalysator wird mit hochdispersem $SiO_2$ im Gewichtsverhältnis 80:20 zu 2,5-mm-Strängen verstrangt, bei 110 °C/16 h getrocknet und bei 500 °C/24 h calciniert.

Katalysator I

Kommerziell erhältlicher L-Zeolith (Baylith L®) wird mit Verformungshilfsmitteln zu 2-mm-Strängen verformt. Nach Trocknen bei 110 °C/16 h und Calcination bei 500 °C/16 h liegt der fertige Katalysator I vor.

Katalysator J

$AlPO_4$-5 (APO-5) wird synthetisiert, indem man 200 g 98 %ige Phosphorsäure und 136 g Boehmit in 335 g Wasser löst bzw. suspendiert, hierzu 678 g einer 30 %igen wäßrigen Tetrapropylammoniumhydroxid-Lösung zugibt und diese Mischung in einem Rührautoklaven bei 150 °C in 43 h unter autogenem Druck umsetzt. Nach Abfiltrieren wird das kristalline Material bei 120 °C getrocknet und 500 °C/16 h calciniert. Das so synthetisierte $AlPO_4$-5 enthält 45,5 Gew.% $Al_2O_3$ und 46,5 Gew.% $P_2O_5$. Dieses Material wird mit Pseudo-Boehmit im Massenverhältnis 60:40 zu 2-mm-Strängen verformt, abermals bei 120 °C getrocknet und bei 500 °C/16 h calciniert.

Katalysator K

$AlPO_4$-9 (APO-9) wird synthetisiert, indem man 200 g 98 %ige Phosphorsäure und 136 g Boehmit in

400 g Wasser löst bzw. suspendiert, hierzu eine wäßrige Lösung aus 112 g Diazabicyclo-2,2,2-octan (DABCO) und 320 g $H_2O$ zugibt und diese Mischung in einem Rührautoklaven bei 200 °C während 336 h unter autogenem Druck umsetzt. Nach Abfiltrieren wird das kristalline Material bei 120 °C getrocknet und bei 500 °C/16 h calciniert. Das so synthetisierte $AlPO_4$-9 enthält 49,0 Gew.% $P_2O_5$, 37,1 Gew.% $Al_2O_3$. Dieses Material wird mit Verstrangungshilfsmitteln zu 3-mm-Strängen verformt, abermals bei 120 °C getrocknet und bei 500 °C/6h calciniert.

Katalysator I

Siliciumphosphat-5 (SAPO-5) wird aus einer Mischung aus 200 g 98 %iger Phosphorsäure, 136 g Boehmit, 60 g Kieselöl (30 %ig), 287 g Tripropylamin und 587 g $H_2O$ hergestellt. Diese Mischung wird bei 150 °C während 1168 h unter autogenem Druck umgesetzt. Nach Filtration wird das kristalline Produkt bei 120 °C getrocknet und bei 500 °C calciniert. SAPO-5 enthält 49,8 Gew.% $P_2O_5$, 33,0 Gew.% $Al_2O_3$, 6,2 Gew.% $SiO_2$. SAPO-5 wird mit einem Verstrangungshilfsmittel zu 3-mm-Strängen verformt, bei 120 °C getrocknet und bei 500 °C calciniert.

Katalysator M

$BPO_4$ wird hergestellt, indem man 49 g $H_3BO_3$ mit 117 g $H_3PO_4$ (75 %ig) in einem Kneter zusammengibt, überschüssiges Wasser abdampft und das Umsetzungsprodukt zu 3-mm-Strängen verformt. Diese Stränge werden bei 100 °C getrocknet und bei 350 °C calciniert. Katalysator D enthält 8,77 Gew.% B und 28,3 Gew.% P.

Katalysator N

Katalysator N ist ein gefälltes Aluminiumphosphat, das durch Fällung aus $Al(NO_3)_3$-$H_3PO_4$-Lösung mit $NH_3$ bei pH = 6 bis 7 erhalten wird. Nach Abfiltrieren des Niederschlags wird bei 110 °C getrocknet und bei 500 °C calciniert. Katalysator N enthält 28,5 Gew.% Al und 13,2 Gew.% P.

Katalysator O

Im Handel erhältliches Zirkonphosphat (CZP 100®) wird mit Verformungshilfsmitteln zu 2-mm-Strängen verformt, bei 100 °C getrocknet und bei 500 °C/16 h calciniert.

Katalysator P

$CePO_4$ wird durch Fällung aus 52 g $Ce(NO_3)_3$ x 6 $H_2O$ und 56 g $NaH_2PO_4$ x 2 $H_2O$ erhalten. Nach der Filtration wird das Material zu Strängen verformt, bei 120 °C getrocknet und bei 450 °C calciniert. Katalysator P enthält 47,1 Gew.% Ce und 12,7 Gew.% P.

Katalysator Q

$SiO_2$ im Handel erhältlich unter D 11-10®.

Katalysator R

100 g $SiO_2$-Stränge (D 11-10®) werden 280 ml 0,1 n HF und 80 ml $H_2O$ unter Rückfluß 1 h behandelt. Danach wird das Material neutral gewaschen, bei 110 °C getrocknet und bei 500 °C/5 h calciniert.

Katalysator S

$Al_2O_3$ im Handel erhältlich unter D 10-10®.

Katalysator T

D 10-10® wird mit $H_3BO_3$ imprägniert, getrocknet bei 110 °C und bei 500 °C/5 h calciniert. Der Katalysator T setzt sich zusammen aus 85 % $Al_2O_3$ und 15 % $B_2O_3$.

Katalysator U

Katalysator U wird erhalten, indem man D 10-10® $Al_2O_3$ mit 85 %iger $H_3PO_4$ 30 min behandelt, danach 130 °C/2 h trocknet und bei 540 °C/2 h calciniert. Der P-Gehalt beträgt 4,9 Gew.%.

Katalysator V

$TiO_2$ P 25® wird zu 2-mm-Strängen verformt, bei 110 °C getrocknet und bei 500 °C/16 h calciniert.

Katalysator W

200 g Katalysator V werden mit 600 ml 15 %iger HCl bei 80 °C/1 h behandelt. Danach wird das Material Cl-frei gewaschen, bei 110 °C getrocknet und bei 600 °C/1 h calciniert.

Katalysator X

Kommerziell erhältliches Nioboxydhydrat wird mit hochdispersem $SiO_2$ zu 2,5-mm-Strängen verformt, bei 130 °C/16 h getrocknet und bei 300 °C/4 h calciniert.

Katalysator Y

Katalysator Y wird mit wäßriger Palladiumnitrit-Lösung imprägniert. Nach Trocknung bei 130 °C/2 h und Calcination bei 540 °C/2 h beträgt der Pd-Gehalt 1,5 Gew.%.

Katalysator Z

Katalysator Z ist ein kommerziell erhältlicher Y-Zeolith, der mit einer Pd-Nitrat-Lösung ionenausgetauscht ist. Der Pd-Gehalt beträgt 0,5 Gew.%.

Beispiele

Beispiele 1 bis 50

Die Reaktionen in der Gasphase wurden unter isothermen Bedingungen in einem Rohrreaktor (Wendel, 0,6 cm Innendurchmesser, 90 cm Länge) mindestens 6 Stunden lang durchgeführt. Die Reaktionsprodukte wurden durch übliche Methoden abgetrennt und charakterisiert. Die quantitative Bestimmung der Reaktionsprodukte und der Ausgangsprodukte erfolgte gaschromatographisch.

Beispiel 1

Eine wäßrige Piperazin-Lösung (10 Gew.% Piperazin) wird gasförmig bei 400 °C und einer Belastung

WHSV = 3 $h^{-1}$ über Katalysator A in voran beschriebener Apparatur umgesetzt. Der Reaktionsaustrag enthält 53,7 Gew.% Piperazin, 18,4 Gew.% DABCO, 4,3 Gew.% N-Methylpiperazin und 1,3 Gew.% N-Ethylpiperazin.

Beispiel 2

Die Versuchsdurchführung erfolgt wie bei Beispiel 1, jedoch wird an Katalysator G umgesetzt. Der Reaktionsaustrag enthält 23,2 Gew.% Piperazin, 30,5 Gew.% DABCO, 16,2 Gew.% N-Methylpiperazin, 3,9 Gew.% N-Ethylpiperazin und 3,5 Gew.% Methylpiperazin.

Beispiele 3 bis 34

Die Versuchsdurchführung erfolgt analog Beispiel 1. Die Ergebnisse sind in der nachfolgenden Tabelle 1 aufgeführt.

Tabelle 1: 1,4-Diazabicyclo-2,2,2-octan (DABCO) aus Piperazin

| Beispiel | 3 | 4 | 5 | 6 | 7 | 8 | 9 | 10 | 11 | 12 | 13 | 14 |
|---|---|---|---|---|---|---|---|---|---|---|---|---|
| Katalysator | A | A | A | A | A | A | B | B | C | D | E | F |
| Temp., °C | 400 | 400 | 300 | 400 | 300 | 400 | 400 | 400 | 400 | 400 | 400 | 400 |
| WHSV $h^{-1}$ | 2,5 | 1,5 | 3 | 3 | 3 | 2,5 | 2,5 | 2,5 | 2,5 | 2,5 | 2,5 | 2,5 |
| wäßr. Lösung % | 25 | 50 | 50 | 50 | 50 | 25 | 25 | 25 | 25 | 25 | 25 | 25 |
| Piperazin/[2] Ethanol | 1:1 | 1:1 | 1:1 | 1:2 | 1:2 | 1:6 | 1:1 | 1:2 | 1:2 | 1:1 | 1:1 | 1:1 |
| Umsatz %[1] | 57,1 | 37,0 | 12,0 | 36,5 | 9,8 | 62,4 | 60,4 | 68,4 | 68,4 | 48,6 | 52,4 | 67,2 |
| Selekt. %[1] | | | | | | | | | | | | |
| DABCO | 64,0 | 81,8 | 74,4 | 78,9 | 91,2 | 65,3 | 74,0 | 71,0 | 50,2 | 76,5 | 78,9 | 78,6 |
| N-Methyl-piperazin | 17,9 | 8,4 | - | 5,5 | 0,4 | 9,9 | 10,3 | 8,8 | 29,8 | 7,4 | 6,3 | 9,8 |
| N-Ethyl-piperazin | 5,8 | 9,7 | 3,3 | 7,7 | 1,8 | 14,9 | 5,5 | 6,4 | 6,2 | 7,4 | 8,6 | 5,5 |

1) bezogen auf Piperazin
2) Molverhältnis

Tabelle 1 (Fortsetzung)

| Beispiel | 15 | 16 | 17 | 18 | 19 | 20 | 21 | 22 | 23 | 24 | 25 | 26 |
|---|---|---|---|---|---|---|---|---|---|---|---|---|
| Katalysator | F | F | G | H | I | J | K | L | M | N | O | P |
| Temp., °C | 400 | 400 | 400 | 400 | 400 | 400 | 400 | 400 | 400 | 400 | 400 | 400 |
| WHSV $h^{-1}$ | 2,5 | 2,5 | 2,5 | 1,5 | 1,5 | 1,5 | 1,5 | 1,5 | 1,5 | 2,5 | 2,5 | 2,5 |
| wäßr. Lösung % | 25 | 25 | 25 | 25 | 25 | 25 | 25 | 25 | 25 | 25 | 25 | 25 |
| Piperazin/[2] Ethanol | 1:2 | 1:6 | 1:1 | 1:1 | 1:1 | 1:1 | 1:1 | 1:1 | 1:1 | 1:1 | 1:1 | 1:1 |
| Umsatz %[1] | 72,9 | 75,1 | 85,6 | 28,6 | 19,5 | 51,2 | 58,9 | 65,9 | 76,2 | 69,2 | 62,6 | 63,4 |
| Selekt. %[1] | | | | | | | | | | | | |
| DABCO | 75,9 | 73,3 | 71,0 | 45,7 | 47,6 | 61,7 | 64,2 | 67,8 | 44,3 | 47,5 | 44,5 | 36,5 |
| N-Methyl-piperazin | 9,5 | 5,4 | 8,8 | 19,2 | 20,2 | 13,9 | 9,3 | 8,7 | 5,0 | 11,7 | 7,8 | 18,2 |
| N-Ethyl-piperazin | 5,8 | 11,0 | 9,73 | 27,3 | 22,0 | 12,8 | 5,4 | 7,6 | 6,2 | 5,3 | 33,9 | 8,8 |

1) bezogen auf Piperazin

2) Molverhältnis

Tabelle 1 (Fortsetzung)

| Beispiel | 27 | 28 | 29 | 30 | 31 | 32 | 33 | 34 |
|---|---|---|---|---|---|---|---|---|
| Katalysator | Q | R | S | T | U | V | W | X |
| Temp., °C | 400 | 400 | 400 | 400 | 400 | 400 | 400 | 400 |
| WHSV $h^{-1}$ | 2,5 | 2,5 | 2,5 | 2,5 | 2,5 | 2,5 | 2,5 | 2,5 |
| wäßr. Lösung % | 25 | 25 | 25 | 25 | 25 | 25 | 25 | 25 |
| Piperazin/[2] Ethanol | 1:1 | 1:1 | 1:1 | 1:1 | 1:1 | 1:1 | 1:1 | 1:1 |
| Umsatz %[1] | 57,7 | 68,4 | 45,7 | 69,9 | 72,3 | 40,3 | 49,2 | 80,4 |
| Selekt. %[1] | | | | | | | | |
| DABCO | 33,2 | 28,1 | 37,9 | 45,9 | 49,3 | 52,3 | 51,3 | 47,3 |
| N-Methyl-piperazin | 12,3 | 11,0 | 6,1 | 13,0 | 11,3 | 8,4 | 7,9 | 1,2 |
| N-Ethyl-piperazin | 8,9 | 11,0 | 15,9 | 11,6 | 15,8 | 18,9 | 16,2 | 12,6 |

1) bezogen auf Piperazin
2) Molverhältnis

Beispiel 35

Ein Gemisch aus Piperazin und Ethanol im moralen Verhältnis 1 : 2 wird in 25%iger wäßriger Lösung zusammen mit 8 l/h $N_2$ und 4 l/h Luft bei 400 °C und WHSV = 2,5 $h^{-1}$ an Katalysator Y zugesetzt. Der Umsatz beträgt 91,6 %, die Selektivität für DABCO 90,2 %, für N-Methylpiperazin 0,5 % und für N-Ethylpiperazin 0,4 %.

Beispiel 36

Es wird wie in Beispiel 35 verfahren, jedoch wird Katalysator Z eingesetzt und bei 300 °C gearbeitet. Der Umsatz beträgt 76,9 %, die Selektivität für DABCO 86,3 %, für N-Methylpiperazin 2,1 % und für N-Ethylpiperazin 5,4 %.

Beispiel 37

Piperazin wird in THF (molares Verhältnis 1 : 4) verdampft und bei 400 °C und WHSV = 1,5 $h^{-1}$ an Katalysator G umgesetzt. Der Umsatz des Piperazins beträgt 79,5 % und die DABCO-Selektivität 65,3 %.

Beispiel 38

Beispiel 38 beschreibt einen Langzeitversuch am Katalysator A. Die Umsetzung wurde in einem Rohrreaktor (Innendurchmesser 2 cm), der mit 100 g Katalysator A gefüllt war, isotherm bei 400 °C und WHSV = 1 $h^{-1}$ durchgeführt. Es wurde ein Gemisch aus Piperazin/Ethanol (1 : 1 molar) in 25 % wäßriger Lösung und 10 l $N_2$/h eingesetzt. Nach dem 5. Tag wurde eine Regenerierung durchgeführt. Der Reaktionsverlauf ist in Tabelle 2 beschrieben.

Tabelle 2

| DABCO aus Piperazin/Ethanol | | | | | | | | | | |
|---|---|---|---|---|---|---|---|---|---|---|
| Langzeitversuche bei 400 °C, WHSV = ca. 1 $h^{-1}$, 1 : 1 molar, | | | | | | | | | | |
| 25 % wäßrige Lösung, 10 l $N_2$/h | | | | | | | | | | |
| Katalysator | A | | | | | regenerierter A | E | | | |
| Laufzeit | 1. Tag | 2. Tag | 3. Tag | 4. Tag | 5. Tag | 1. Tag | 1. Tag | 2. Tag | 3. Tag | 4. Tag |
| DABCO[1)] | 34,9 | 29,1 | 28,1 | 25,7 | 25,5 | 35,0 | 40,4 | 31,6 | 28,4 | 28,1 |
| Piperazin[1)] | 50,8 | 58,4 | 58,3 | 60,3 | 60,6 | 49,0 | 40,1 | 48,2 | 50,0 | 50,4 |
| N-Methyl-piperazin | 2,9 | 2,2 | 1,8 | 1,5 | 2,0 | 3,3 | 3,9 | 2,5 | 2,0 | 1,9 |
| N-Ethyl- | 3,6 | 3,0 | 2,7 | 2,5 | 2,6 | 3,2 | 4,2 | 3,3 | 3,3 | 2,9 |

1) Gehalt in % im Austrag

2) Katalysator A nach 5 Tagen Laufzeit regeneriert bei 500 °C mit Luft 4 h

Tabelle 3

| DABCO aus Piperazin | | | | | | | | | | | |
|---|---|---|---|---|---|---|---|---|---|---|---|
| Beispiel | 40 | 41 | 42 | 43 | 44 | 45 | 46 | 47 | 48 | 49 | 50 |
| Katalysator | A | A | E | G | G | A | A | B | E | G | G |
| Temp., °C | 300 | 400 | 400 | 300 | 400 | 300 | 400 | 400 | 400 | 300 | 400 |
| WHSV $h^{-1}$ | 3 | 3 | 3 | 3 | 3 | 3 | 3 | 2 | 3 | 2 | 2 |
| wäßr. Lösung % | 25 | 25 | 25 | 25 | 25 | 25 | 25 | 25 | 25 | 25 | 25 |
| Piperazin/ | tert. Butanol | | | | | Diethylenglykol | | | | | |
| Alkohol | 1 : 1 molar | | | | | 1 : 1 molar | | | | | |
| Umsatz % | 13,3 | 61,4 | 51,7 | 25,0 | 85,5 | 26,8 | 58,8 | 59,4 | 62,8 | 26,2 | 73,7 |
| Selekt. % | | | | | | | | | | | |
| DABCO | 78,2 | 78,0 | 88,2 | 84,0 | 70,1 | 45,3 | 56,7 | 66,5 | 62,2 | 46,8 | 51,6 |
| N-Methy-piperazin | - | 10,2 | 7,1 | 4,0 | 6,9 | 1,1 | 9,2 | 9,5 | 11,5 | 5,3 | 9,3 |
| N-Ethyl-piperazin | - | 5,2 | 1,3 | 1,2 | 2,1 | 2,2 | 3,2 | 7,3 | 3,8 | 19,8 | 12,8 |

## Ansprüche

Verfahren zur Herstellung von 1,4-Diazabicyclo-2,2,2-octanen der allgemeinen Formel I

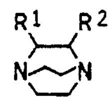

(I),

in der $R^1$ und $R^2$ unabhängig voneinander für Wasserstoff, $C_1$- bis $C_4$-Alkyl oder $C_2$- bis $C_4$-Alkenyl stehen, dadurch gekennzeichnet, daß man Piperazine der allgemeinen Formel II

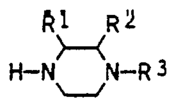

(II),

in der $R^1$ und $R^2$ die oben genannten Bedeutungen haben und $R^3$ für Wasserstoff oder $C_1$- bis $C_8$-Alkyl steht, in Gegenwart von Heterogenkatalysatoren bei Temperaturen von 50 bis 600 °C und Drücken von 0,01 bis 50 bar, gegebenenfalls unter Mitverwendung eines geeigneten Lösungs- oder Verdünnungsmittels, umsetzt.